# EUROPÄISCHE PATENTANMELDUNG

(11) **EP 1 917 863 A1**
(43) Veröffentlichungstag der Anmeldung: **07.05.2008**
(21) Anmeldenummer: 07020519.0
(22) Anmeldetag: 19.10.2007
(51) Int. Cl.: A23D 9/02, C11B 3/14, A23L 1/30, A61K 8/92, A61K 31/232

(54) **Fettes Öl aus Perillasamen mit verbesserten Eigenschaften**

(30) Priorität: 03.11.2006 DE 102006052011
(71) Anmelder: Dr. Willmar Schwabe GmbH & Co. KG, 76227 Karlsruhe (DE)
(72) Erfinder: Herrmann, Joachim, Dr., 68789 St. Leon-Rot (DE); Lang, Friedrich, Dr., 76767 Hagenbach (DE)
(74) Vertreter: Adam, Holger

(57) **Zusammenfassung**

Die vorliegende Erfindung betrifft ein fettes Öl aus Perillasamen mit verbesserten Eigenschaften, besonders geeignet zur Verwendung als Lebensmittel, Arzneimittel oder Kosmetikum. Die vorliegende Erfindung betrifft insbesondere ein fettes Öl aus Perillasamen mit gleichzeitig sehr geringem Gehalt an Perillaketon und trans-Fettsäuren sowie ein Verfahren zur Herstellung dieses fetten Öls.

## Beschreibung

Die vorliegende Erfindung betrifft ein fettes Öl aus Perillasamen mit verbesserten Eigenschaften, besonders geeignet zur Verwendung als Lebensmittel, Arzneimittel oder Kosmetikum. Die vorliegende Erfindung betrifft insbesondere ein fettes Öl aus Perillasamen mit gleichzeitig sehr geringem Gehalt an Perillaketon und trans-Fettsäuren sowie ein Verfahren zur Herstellung dieses fetten Öls.

Die Pflanze Perilla frutescens (Schwarznessel, englisch Beefsteak plant) aus der Familie der Lamiaceae ist in Asien als Kulturpflanze weit verbreitet. Als Rohstoff für Lebensmittel und Arzneimittel werden verschiedene Pflanzenteile verwendet, von denen die Samen die größte wirtschaftliche, ernährungsphysiologische und medizinische Bedeutung haben. Die Samen enthalten 35 - 45 % fettes Öl mit besonders hohem Gehalt an Triglyzeriden mit mehrfach ungesättigten Fettsäuren. Dieses Öl enthält mehr als 50 % Linolensäure (in Form von Triglyzeriden), eine ω-3-Fettsäure mit vielfältigen, für Menschen und Tiere gesundheitsfördernden Eigenschaften. Es wird vor allem in Südostasien aufgrund seines ausgeprägten Geschmacks als Speiseöl verwendet ("Lipid composition and nutritional and physiological roles of perilla seed and its oil", H.-S. Shin, Kap. 9, aus "Perilla, The Genus Perilla", Ed. H.-C. Yu, K. Kosuna, M. Haga, harwood academic publishers, Amsterdam, 1997).

Die Perillapflanze enthält außerdem ätherisches Öl das aufgrund des hohen Gehaltes an wohlriechendem Rosenfuran in der Parfümindustrie verwendet wird. Dieses ätherische Öl (englisch "essential oil") besteht aus Mono- und Sesquiterpenen, Phenylpropanoiden und Furylketonen und ist zu unterscheiden von dem aus Triglyceriden bestehenden, für Ernährungszwecke verwendeten fetten Öl aus den Perillasamen.

Toxikologisch relevant ist bei Perilla frutescens in erster Linie Perillaketon (1(3-Furanyl)-4-methyl-1-pentanon). Perillaketon ist eine toxische Substanz mit einer LD50 i.p. von 2,5 bzw. 6 mg/kg in weiblichen bzw. männlichen Mäusen und 10 mg/kg in männlichen Ratten (Merck Index 11th Edition, 1989, S. 1136) und führt zur Bildung von Lungenödemen ("Perilla Ketone: A Potent Lung Toxin from the Mint Plant, Perilla frutescens Britton", B.J. Wilson, J.E. Garst, Science, Vol. 197, 1977, S. 573 - 574). Der in der Pflanze außerdem vorkommende Perillaaldehyd soll allergieauslösende Wirkung besitzen.

Fettes Öl aus Pflanzensamen wird im Allgemeinen durch Pressung gewonnen. Anschließend kann eine Raffination erfolgen, um die Qualität, die Lagerfestigkeit, das Aussehen und den Geschmack des Öles zu verbessern. Die Raffination von Pflanzenölen kann die folgenden Schritte umfassen: Klärung (zur Beseitigung der in Suspension befindlichen Fremdstoffe) Entschleimung (zur Beseitigung der Schleim- und Eiweißstoffe), Entsäuerung (zur Beseitigung von freien Fettsäuren), Bleichung (zur Beseitigung der Farbstoffe), Desodorierung (zur Beseitigung der Geruchs- und Geschmackstoffe) Winterisierung (zur Beseitigung der Wachse und anderer Stoffe mit höherem Schmelzpunkt) ("Gewinnung und Verarbeitung der pflanzlichen Fette und Öle", Marek Singer, 1992 Verlag für chemische Industrie, Seite 204).

Die aus dem Jahre 1946 stammende US-Patentschrift 2,407,616 beschreibt ein Verfahren zur Desodorisierung von relativ nichtflüchtigem fetten Material das freie Fettsäuren und Geruchsstoffe enthält, umfassend das Durchleiten von Dampf durch die Masse des fetten Materials bei einer Temperatur von mindestens 200° Fahrenheit (93°C) im Vakuum und Entfernen der Dämpfe in ausreichender Menge, um im Wesentlichen die freien Fettsäuren zu verringern und den Geruch des flüssigen Materials zu verbessern, wobei den Dämpfen oberhalb der Masse des fetten Materials Hitze zugeführt wird, derart, dass ein ausreichender Anteil der Dampfzone bei einer Temperatur oberhalb des Kondensationspunktes des Dampfes gehalten wird, um zu verhindern, dass Kondensat bei der Behandlung wieder in das flüssige fette Material gelangt. Gemäß den abhängigen Ansprüchen handelt es sich bei dem fetten Material vorzugsweise um Schweinefett und Baumwollsamenöl. Perillaöl ist lediglich in einer sehr umfangreichen Liste von Materialien in der Beschreibung genannt, jedoch nicht in den Beispielen angegeben. Diese Druckschrift bezieht sich auch nicht auf die Entfernung von Perillaketon aus Perillasamenöl.

Zudem stellt sich gerade bei Perillasamenöl nicht das in der US 2,407,616 beschriebene Problem der Desodorisierung.

Die Verfahrenschritte der Raffination müssen bei Fetten, die einen hohen Anteil an mehrfach ungesättigten Fetten enthalten und für den Verzehr gedacht sind, sehr sorgfältig durchgeführt werden, um die Bildung der unerwünschten trans-Fettsäuren zu vermeiden. In vielen Humanstudien wurde gezeigt, dass die Menge an trans-Fettsäuren in der Ernährung mit einer Erhöhung der Serumkonzentration von LDL-Cholesterol und einer Erniedrigung des HDL-Cholesterols korreliert. Diese Serumkonzentrationen werden wiederum ursächlich mit einem erhöhten Risiko für die koronare Herzkrankheit in Verbindung gebracht (Opinion of the Scientific Panel on Dietetic Products, Nutrition and Allergies on a request from the Commission related to the presence of trans fatty acids in foods and the effect on human health of the consumption of trans fatty acids. Request N° EFSA-Q2003-022, 8 July 2004).

Bei Perillasamenöl sind gemäß der Lehre im Stand der Technik die meisten dieser Herstellungsschritte nicht erforderlich. Es wird durch mechanische Pressung der Samen gewonnen und mittels einfacher Filtration gereinigt ("Lipid composition and nutritional and physiological roles of perilla seed and its oil", H.-S. Shin, Kap. 9 aus "Perilla, The Genus Perilla", Ed. H.-C. Yu, K. Kosuna, M. Haga, harwood academic publishers, Amsterdam, 1997).

Das fette Öl der Perillasamen wird in großem Umfang als Lebensmittel verzehrt und die Anwesenheit.des toxischen Perillaketons in der Pflanze, dem ätherischen Öl der Pflanze und in Öl aus gerösteten Perillasamen ist beschrieben ("The effects of roasting temperatures on the formation of headspace volatile compounds in Perilla seed oil"; S.J. Kim, H.N. Yoon, J. S. Rhee, JOACS (Journal of the American Oil Chemists Society), Vol. 77, 4, 2000, S. 451 - 456.) Dennoch machen die Lieferanten in ihren Qualitätsbeschreibungen den so genannten Spezifikationen keine Angaben, ob und wie viel toxisches Perillaketon im fetten Öl enthalten ist. Die Analyse von Ölchargen verschiedener Lieferanten ergab den Befund, dass in kommerziell erhältlichem fettem Öl aus Perillasamen signifikante Mengen an Perillaketon enthalten sind (siehe Tabelle 1). Der als allergen beschriebene Perillaaldehyd konnte in keiner der Ölchargen nachgewiesen werden.

**Tabelle 1: Gehalte von Perillaketon in kommerziell erhältlichem fettem Öl aus Perillasamen (Analysen-Methode: Headspace GC-MS, DB-Wax-Säule, Säulenlänge 60 m, Ofentemperatur: 80 °C)**

| Charge des Öls | Gehalt an Perillaketon [ppm]* | Gehalt an Perillaaldehyd [ppm]* |
|---|---|---|
| A | 58 | < 5 |
| B | 269 | < 5 |
| C | 49 | < 5 |
| D | 40 | < 5 |
| E | 45 | < 5 |

| | | |
|---|---|---|
| * Analytische Nachweisgrenze für Perillaketon und Perillaaldehyd 5 ppm | | |

Aufgabe der Erfindung ist die Bereitstellung eines fetten Öles aus Perillasamen, das die gesundheitsfördernde, in der Literatur beschriebene natürliche Fettsäurezusammensetzung mit einem hohen Gehalt an mehrfach ungesättigten w-3-Fettsäuren besitzt und frei oder nahezu frei von gesundheitsschädlichem Perillaketon ist und verglichen mit dem auf herkömmliche Weise durch Pressung und Filtration hergestellten Öl einen unveränderten oder nahezu unveränderten Gehalt an trans-Fettsäuren aufweist.

Diese Aufgabe wird erfindungsgemäß gelöst durch ein fettes Öl aus Perillasamen mit einem Gehalt von höchstens 20 ppm, vorzugsweise höchstens 10 ppm, insbesondere höchstens 5 ppm Perillaketon und einem Gehalt von höchstens 2 %, vorzugsweise höchstens 1%, an trans-Fettsäuren.

Das erfindungsgemäße fette Öl aus Perillasamen wird durch ein Verfahren erhalten, bei dem durch Pressung, Filtration, Entschleimung, Neutralisation und Bleichung hergestelltes fettes Öl aus Perillasamen bei etwa 160°C bis etwa 210°C, vorzugsweise etwa 180°C bis etwa 200°C für eine Dauer von etwa 180 bis etwa 90 Minuten, vorzugsweise etwa 160 bis etwa 130 Minuten mit Wasserdampf behandelt wird. Besonders bevorzugt ist die Behandlung bei etwa 200°C für etwa 130 Minuten und bei etwa 180°C für etwa 160 Minuten.

Das erfindungsgemäße fette Öl aus Perillasamen ist besonders gut geeignet zur Verwendung als Lebensmittel, Arzneimittel und Kosmetikum zur Anwendung auf der Haut.

Vergleichsbeispiele 1 bis 3 (Tabelle 2): Herstellung eines Perillasamen-Rohöles unter herkömmlichen Bedingungen durch Pressung und Filtration (Herstellungsschritt 1; Vergleichsbeispiel 1) und Weiterverarbeitung mit üblichen, bei Perillasamenöl herkömmlicherweise nicht angewendeten Raffinationsverfahren (Herstellungsschritte 2 und 3; Vergleichsbeispiele 2 und 3) .

Zur Gewinnung des Öls aus den Samen wurde eine hydraulische Schneckenpresse verwendet, wie sie heute in Ölmühlen praktisch ausschließlich eingesetzt werden. Anschließend wurde das Öl mit einer herkömmlichen Rahmen- und Kammerfilterpresse filtriert, um feste Anteile anorganischer und organischer Stoffe, wie Schleimstoffe, Eiweißstoffe und Wachse zu entfernen (Vergleichsbeispiel 1). Durch Zugabe von verdünnter Phosphorsäure wurde das Öl entschleimt und in der gleichen Anlage durch Zugabe von Natronlauge entsäuert bzw. neutralisiert. Das eingebrachte Wasser wurde durch Trocknung entfernt. Durch Zugabe von natürlicher Bleicherde und Aktivkohle wurden Farbstoffe und andere Verunreinigungen entfernt (Vergleichsbeispiel 2). Anschließend wurde das erhaltene teilraffinierte Öl bei erhöhter Temperatur mit Wasserdampf behandelt (Vergleichsbeispiel 3).

| Herstellungsschritt | erhaltenes Produkt | Gehalt an Perillaketon [ppm] | Gehalt an trans-Fettsäuren [%] |
|---|---|---|---|
| 1 (Vergleichs- | Gepresstes und filtriertes Öl aus Perilla-Samen | 77 | 0,5 |

| beispiel 1) | | | |
|---|---|---|---|
| 2 (Vergleichsbeispiel 2) | Ausgehend von Schritt 1 entschleimtes, neutralisiertes und gebleichtes Öl aus Perillasamen | 52 | 0,4 |
| 3 (Vergleichsbeispiel 3) | Ausgehend von Schritt 2 mit Wasserdampf bei 240 °/90 Minuten behandeltes Öl aus Perillasamen | nicht nachweisbar (< 5) | 8,2 |

Das durch Pressung und Filtration von Perilla-Samen auf herkömmliche Weise hergestellte fette Öl hat einen hohen Gehalt an toxischem Perillaketon und einen niedrigen Gehalt an gesundheitsschädlichen trans-Fettsäuren. Durch weitere Raffinationsschritte wird der Gehalt an Perillaketon etwas verringert. Durch die anschließende Behandlung mit Wasserdampf unter den genannten Bedingungen kann zwar der Gehalt an Perillaketon unter die analytische Nachweisgrenze gebracht werden, das erhaltene Öl hat aber, verglichen mit dem auf herkömmliche Weise durch Pressung und Filtration hergestellten Öl, einen sehr hohen Gehalt an trans-Fettsäuren.

### Beispiele 1 und 2 (Tabelle 3): Herstellung eines Perillasamenöls unter erfindungsgemäßen Bedingungen.

| Herstellungsschritt | Erhaltenes Produkt | Gehalt an Perillaketon [ppm] | Gehalt an trans-Fettsäuren [%] |
|---|---|---|---|
| 1 - 3 (Beispiel 1) | Gepresstes, filtriertes, entschleimtes, neutralisiertes, gebleichtes und mit Wasserdampf behandeltes (200 °/130 min) Öl aus Perillasamen | nicht nachweisbar (< 5) | 1,0 |
| 1 - 3 | Gepresstes, filtriertes, entschleimtes, neutralisiertes, | nicht nachweisbar (< 5) | 0,6 |
| (Beispiel 2) | gebleichtes und mit Wasserdampf behandeltes (180 °/160 min) Öl aus Perillasamen | | |

Die erfindungsgemäße Aufgabe wird gelöst, in dem das auf herkömmliche Weise gepresste und filtrierte und zusätzlich entschleimte, neutralisierte, gebleichte Perillasamenöl mit heißem Wasserdampf für eine Dauer von etwa 180-90 Minuten bei etwa 160°C bis etwa 210°C, vorzugsweise für eine Dauer von 160-130 Minuten bei etwa 180°C bis etwa 200°C behandelt wird. Vorzugsweise erfolgt die Behandlung mit Wasserdampf bei etwa 200°C für etwa 130 Minuten und insbesondere bei etwa 180°C für etwa 160 Minuten. Nur das erfindungsgemäße Herstellungsverfahren liefert ein Öl mit einem Gehalt von höchstens 2 Gewichtsprozent, vorzugsweise höchstens 1 Gew.-% an trans-Fettsäuren mit einem Gehalt von höchstens 20 ppm, vorzugsweise höchstens 10 ppm und insbesondere höchstens 5 ppm Perillaketon.

## Patentansprüche

1. Fettes Öl aus Perillasamen mit einem Gehalt von höchstens 20 ppm an Perillaketon und einem Gehalt von höchstens 2 Gew.-% an trans-Fettsäuren.

2. Fettes Öl aus Perillasamen nach Anspruch 1 mit einem Gehalt von höchstens 10 ppm an Perillaketon und einem Gehalt von höchstens 2 Gew.-% an trans-Fettsäuren.

3. Fettes Öl aus Perillasamen nach Anspruch 1 mit einem Gehalt von höchstens 10 ppm an Perillaketon und einem Gehalt von höchstens 1 Gew.-% an trans-Fettsäuren.

4. Fettes Öl aus Perillasamen nach Anspruch 1 mit einem Gehalt von höchstens 5 ppm an Perillaketon und einem Gehalt von höchstens 1 % an trans-Fettsäuren.

5. Verfahren zur Herstellung eines fetten Öles aus Perillasamen gemäß einem der Ansprüche 1 - 4 bei dem durch Pressung, Filtration, Entschleimung, Neutralisation und Bleichung hergestelltes fettes Öl aus Perillasamen bei etwa 160 - 210 °C für eine Dauer von etwa 180 - 90 Minuten mit Wasserdampf behandelt wird.

6. Verfahren zur Herstellung eines fetten Öles aus Perillasamen nach Anspruch 5, wobei die Behandlung mit Wasserdampf bei etwa 180°C bis etwa 200°C für eine Dauer von etwa 160 bis etwa 130 Minuten erfolgt.

7. Verfahren nach Anspruch 6, wobei die Behandlung mit Wasserdampf bei etwa 200°C für etwa 130 Minuten erfolgt.

8. Verfahren nach Anspruch 6, wobei die Behandlung mit Wasserdampf bei etwa 180°C für etwa 160 Minuten erfolgt.

9. Verwendung eines fetten Öles aus Perillasamen gemäß einem der Ansprüche 1 - 4 als Lebensmittel.

10. Verwendung eines fetten Öles aus Perillasamen gemäß einem der Ansprüche 1 - 4 als Arzneimittel.

11. Verwendung eines fetten Öles aus Perillasamen gemäß einem der Ansprüche 1 - 4 als Kosmetikum zur Anwendung auf der Haut.
